(19) Europäisches Patentamt
European Patent Office
Office européen des brevets

(11) Veröffentlichungsnummer: **0 030 620**
**B1**

(12) **EUROPÄISCHE PATENTSCHRIFT**

(45) Veröffentlichungstag der Patentschrift: **13.06.84**

(21) Anmeldenummer: **80106918.8**

(22) Anmeldetag: **10.11.80**

(51) Int. Cl.³: **C 07 D 233/52**, C 07 D 409/12, A 61 K 31/415

(54) **Substituierte 2-Phenylamino-imidazoline-(2), deren Säureadditionssalze, diese enthaltende Arzneimittel und Verfahren zur Herstellung derselben.**

(30) Priorität: **14.12.79 DE 2950345**

(43) Veröffentlichungstag der Anmeldung:
**24.06.81 Patentblatt 81/25**

(45) Bekanntmachung des Hinweises auf die Patenterteilung:
**13.06.84 Patentblatt 84/24**

(84) Benannte Vertragsstaaten:
**AT BE CH DE FR GB IT LI LU NL SE**

(56) Entgegenhaltungen:
**EP - A - 0 007 406**
**EP - A - 0 007 438**
**EP - A - 0 013 252**
**DE - A - 2 457 979**

(73) Patentinhaber: **C.H. BOEHRINGER SOHN**
**Postfach 200**
**D—6507 Ingelheim am Rhein (DE)**

(72) Erfinder: **Stähle, Helmut, Dr.**
**Rotweinstrasse 23**
**D-6507 Ingelheim/Rhein (DE)**
Erfinder: **Köppe, Herbert, Dr.**
**Neuweg 72**
**D-6507 Ingelheim/Rhein (DE)**
Erfinder: **Kummer, Werner, Dr.**
**Georg-Scheuing-Strasse 15**
**D-6507 Ingelheim/Rhein (DE)**
Erfinder: **Lillie, Christian, Dr. Mag.**
**Hansi-Niese-Weg 12**
**A-1130 Wien (AT)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 030 620 B1

**Beschreibung**

Aus der DE—A—24 57 979 sind 2-N-Phenyl-hydroxyamino-imidazoline-(2) bekannt, denen blutdrucksenkende Eigenschaften zugeschrieben werden. Über eine bradycarde Wirkung dieser oder ähnlicher Verbindungen wird in der vorveröffentlichten Literatur nichts berichtet.

Es wurde nun gefunden, daß neue substituierte 2-Phenylamino-imidazoline-(2) der allgemeinen Formel (I)

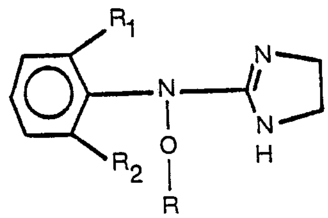

I

sowie deren physiologisch verträgliche Säureadditionssalz wertvolle therapeutische Eigenschaften besitzen.

Die Formel I umfaßt Verbindungen, worin

1) $R_1$ Brom, $R_2$ Fluor und

R $—CH_2—CH{=}CH_2$, $—CH_2—CH{=}CH—CH_3$, $—CH_2—CH_2—CH{=}CH_2$ und

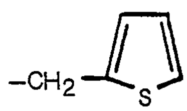

bedeutet

2) Verbindungen, worin

$R_1$ Fluor, $R_2$ Trifluormethyl und

R $—CH_2—CH{=}CH_2$, $—CH_2—CH{=}CH—CH_3$, $—CH_2—C_6H_5$, $—CH_3$, $—C_2H_5$, $-n—C_3H_7$, $-nC_4H_9$

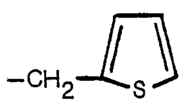 und 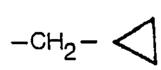

bedeutet

3) Verbindungen, worin

$R_1$ und $R_2$ Brom und

R $—CH_2—CH{=}CH_2$, $—CH_2—CH{=}CH—CH_3$ und

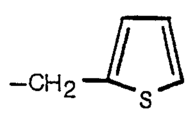

bedeutet

4) eine Verbindung, worin

$R_1$ und $R_2$ Chlor und R

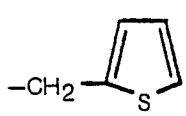

bedeutet sowie

5) eine Verbindung, worin

$R_1$ und $R_2$ Fluor und R $—CH_2—CH{=}CH_2$ bedeutet.

Die Herstellung der Verbindungen der Formel (I) erfolgt durch Umsetzung eines 2-Phenylamino-imidazolins-(2) der allgemeinen Formel (II)

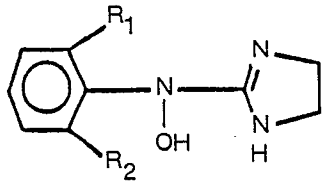

II

in der $R_1$ und $R_2$ die oben genannten Bedeutungen besitzen, mit einem Halogenid der allgemeinen Formel (III)

Hal-R III

worin Hal ein Chlor-, Brom- oder Jodatom bedeutet und R die oben angeführten Bedeutungen hat.

Die Umsetzung erfolgt vorzugsweise in Gegenwart eines polaren oder unpolaren Lösungsmittels bei Raumtemperatur, wobei zweckmäßigerweise die Natriumsalze der Verbindungen der Formel (II) mit den Halogeniden der Formel (III) zur Reaktion gebracht werden. Die speziellen Reaktionsbedingungen hängen in starkem Maße von der Reaktivität der Umsetzungsteilnehmer ab. Es empfiehlt sich, bei der Alkylierung das Halogenid der allgemeinen Formel (III) im Überschuß anzuwenden und die Umsetzung in Gegenwart eines säurebindenden Mittels durchzuführen.

Ausgangsverbindungen der Formel (II) sind literaturbekannt (vgl. DE—A—2 457 979). Verbindungen der Formel (III) lassen sich durch Halogenierung der zugrundeliegenden Alkohole darstellen.

Die erfindungsgemäßen 2-Phenylamino-imidazoline-(2) der allgemeinen Formel (I) können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin.

Die neuen Verbindungen der allgemeinen Formel (I) und deren Säureadditionssalze wirken stark bradycard und sind daher zur Therapie von Coronarerkrankungen geeignet. Die Beeinflussung der Herzfrequenz wurde an Kaninchen und an Spinalratten sowie an intakten, narkotisierten Ratten untersucht.

Die Dosiering liegt bei 0,1 bis 80 mg, vorzugsweise 1 bis 30 mg.

Die Verbindungen der Formel (I) bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

## Herstellungsbeispiele

### Beispiel 1

2-[N-Allyloxy-N-(2-brom-6-fluorphenyl)amino]-2-imidazolin

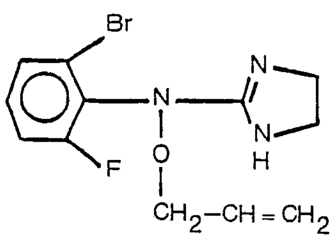

5 g (0,016 Mol) 2-[N-(2-Brom-6-fluorphenyl)-N-(hydroxy)amino]-2-imidazolin-hydrochlorid werden zusammen mit 0,7 g Natrium in 75 ccm absolutem Äthanol gelöst und der Lösung 1,6 ml (etwa 115%) Allylbromid zugefügt.

Die Reaktionsmischung wird sodann 1 Stunde lang bei Raumtemperatur gerührt. Nach dem Einengen löst man den verbleibenden Rückstand in verdünnter Salzsäure (etwa 1 N) und extrahiert die salzsaure Lösung mit Äther (Ätherextrakte werden verworfen). Bei aufsteigenden pH-Werten (stufenweises Alkalisieren mit 2 N Natronlauge) wird fraktioniert mit Äther extrahiert (auch diese Extrakte werden verworfen).

Nunmehr wird mit 5 N NaOH alkalisiert, wobei hochreines 2-[N-Allyloxy-N-(2-brom-6-fluorphenyl)-amino]-2-imidazolin als weiße, amorphe Substanz ausfällt. Ausbeute nach Absaugen, Waschen mit Wasser und Trocknen: 2,95 g (entsprechend 58,3% der Theorie).

Schmelzpunkt: 141 °C.

In analoger Weise wurden die in der folgenden Tabelle zusammengestellten Verbindungen synthetisiert.

| Beispiel Nr. | $R_1$ | $R_2$ | R | Ausbeute (% der Theorie) | Schmelzpunkt (°C) |
|---|---|---|---|---|---|
| 2 | F | $CF_3$ | $—CH_2—CH{=}CH_2$ | 59,2 | 142 |
| 3 | Br | Br | $—CH_2—CH{=}CH_2$ | 62,2 | 167—168 |
| 4 | F | $CF_3$ | $-CH_2-$ 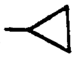 | 7,9 | 117—119 |
| 5 | F | $CF_3$ | $—CH_2—CH{=}CH—CH_3$ | 45,3 | 110—111 |
| 6 | Br | F | $—CH_2—CH{=}CH—CH_3$ | 45,4 | 104 |
| 7 | Br | F | $—CH_2—CH_2—CH{=}CH_2$ | 18,0 | 105 |
| 8 | Br | Br | $—CH_2—CH{=}CH—CH_3$ | 52,8 | 121—122 |
| 9 | F | $CF_3$ | $-CH_2-$ 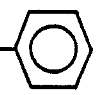 | 60,4 | 113 |
| 10 | F | $CF_3$ | $—C_3H_7$ n | 26,2 | 114 |
| 11 | F | $CF_3$ | $—CH_3$ | 44,5 | 119 |
| 12 | F | $CF_3$ | $—C_2H_5$ | 48,0 | 137 |
| 13 | F | $CF_3$ | $—C_4H_9$ n | 41,8 | 121 |
| 14 | F | F | $—CH_2—CH{=}CH_2$ | 40,0 | 127—128 |
| 15 | Cl | Cl | $-CH_2$ 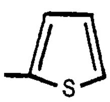 | 35,0 | 131—133 |
| 16 | Br | F | $-CH_2$ 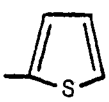 | 44,7 | 136—138 |
| 17 | F | $CF_3$ | $-CH_2$ 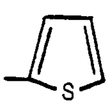 | 40,8 | 97—99 |
| 18 | Br | Br | $-CH_2$ 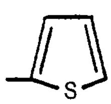 | 28,4 | 125—127 |

Formulierungsbeispiele

Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 |

Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:

Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche für die Vertragsstaaten: BE CH DE FR GB IT LU LI NL SE**

1. Substituierte 2-Phenylamino-imidazoline-(2) der allgemeinen Formel (I)

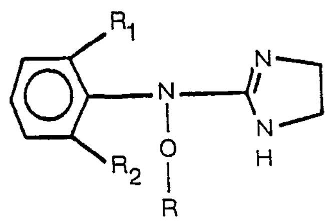

I

worin $R_1$ Brom, $R_2$ Fluor und R $-CH_2-CH=CH_2$, $-CH_2-CH=CH-CH_3$, $-CH_2-CH_2-CH=CH_2$ oder

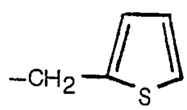

bedeutet sowie deren physiologisch verträgliche Säureadditionssalze.

2. Verbindungen der in Anspruch 1 aufgeführten Formel (I), worin $R_1$ Fluor, $R_2$ Trifluormethyl und

R $-CH_2-CH=CH_2$, $-CH_2-CH=CH-CH_3$, $-CH_2-C_6H_5$, $-CH_3$, $-C_2H_5$, $-nC_3H_7$, $-nC_4H_9$

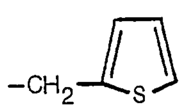 oder 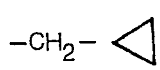

bedeutet sowie deren physiologisch verträgliche Säureadditionssalze.

3. Verbindungen der in Anspruch 1 aufgeführten Formel (I), worin $R_1$ und $R_2$ Brom und R $-CH_2-CH=CH_2$, $-CH_2-CH=CH-CH_3$ oder

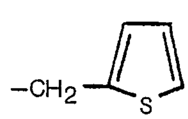

bedeutet sowie deren physiologisch verträgliche Säureadditionssalze.

4. Verbindung der in Anspruch 1 aufgeführten Formel (I), worin $R_1$ und $R_2$ Chlor und R

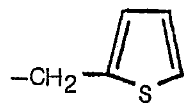

bedeutet sowie dessen physiologisch verträgliche Säureadditionssalze.

5. Verbindung der in Anspruch 1 aufgeführten Formel (I), worin $R_1$ und $R_2$ Fluor und R $-CH_2-CH=CH_2$ bedeutet sowie dessen physiologisch verträglichen Säureadditionssalze.

6. Verfahren zur Herstellung von substituierten 2-Phenylamino-imidazolinen nach Anspruch 1 bis 5, dadurch gekennzeichnet, daß man

ein 2-(N-Phenyl-N-hydroxy-amino)-imidazolin-(2) der allgemeinen Formel (II)

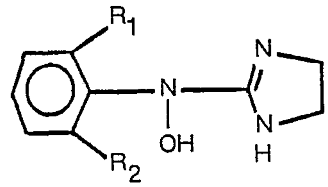
II

worin $R_1$ und $R_2$ die in den Ansprüchen 1 bis 5 genannte Bedeutungen haben, oder dessen Natriumsalz mit einem Halogenid der allgemeinen Formel (III)

$$Hal-R \qquad III$$

worin Hal ein Chlor-, Brom- oder Jodatom bedeutet und R die in den Ansprüchen 1 bis 5 angeführten Bedeutungen hat, umsetzt und daß man gegebenenfalls das erhaltene Endprodukt in ein physiologisch verträgliches Säureadditionssalz überführt.

7. Pharmazeutische Zubereitungen, dadurch gekennzeichnet, daß sie als Wirkstoff eine oder mehrere Verbindungen nach Anspruch 1 bis 5 enthalten.

8. Verfahren zur Herstellung von Arzneimitteln nach Anspruch 7, dadurch gekennzeichnet, daß man eine oder mehrere Verbindungen nach Anspruch 1 bis 5 mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

9. Verbindungen nach Anspruch 1 bis 5 zur Verwendung bei der Bekämpfung von Herz- und Coronarerkrankungen.

**Patentansprüche für den Vertragsstaat: AT**

1. Verfahren zur Herstellung von substituierten 2-Phenylamino-imidazolinen-(2) der allgemeinen Formel (I)

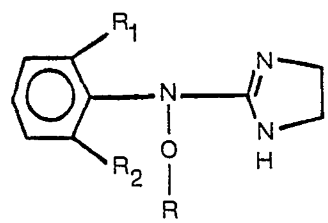
I

worin $R_1$ Brom, $R_2$ Fluor und R $-CH_2-CH=CH_2$, $-CH_2-CH=CH-CH_3$, $-CH_2-CH_2-CH=CH_2$ oder

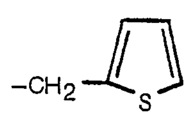

bedeutet sowie von deren physiologisch verträglichen Säureadditionssalzen, dadurch gekennzeichnet, daß man ein 2-(N-Phenyl-N-hydroxyamino)-imidazolin-(2) der allgemeinen Formel (II)

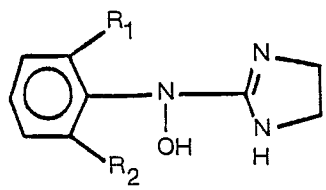
II

worin $R_1$ und $R_2$ die oben genannten Bedeutungen haben, oder dessen Natriumsalz mit einem Halogenid der allgemeinen Formel (III)

Hal—R III

worin Hal ein Chlor-, Brom- oder Jodatom bedeutet und R die oben angeführten Bedeutungen hat, umsetzt und daß man gegebenenfalls das erhaltene Endprodukt in ein physiologisch verträgliches Säureadditionssalz überführt.

2. Verfahren zur Herstellung von substituierten 2-Phenylamino-imidazolinen-(2) der in Anspruch 1 aufgeführten Formel (I), worin $R_1$ Fluor, $R_2$ Trifluormethyl und R $—CH_2—CH{=}CH_2$, $—CH_2—CH{=}CH—CH_3$, $—CH_2—C_6H_5$, $—CH_3$, $—C_2H_5$, $-nC_3H_7$, $-nC_4H_9$,

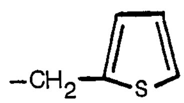 oder 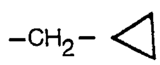

bedeutet und von deren physiologisch verträglichen Säureadditionssalzen, dadurch gekennzeichnet, daß man ein 2-(N-Phenyl-N-hydroxy-amino)-imidazolin-(2) der in Anspruch 1 aufgeführten Formel (II), worin $R_1$ und $R_2$ die oben genannten Bedeutungen haben, oder dessen Natriumsalz mit einem Halogenid der allgemeinen Formel (III)

Hal—R III

worin Hal ein Chlor-, Brom- oder Jodatom bedeutet und R die oben angeführten Bedeutungen hat, umsetzt und daß man gegebenenfalls das erhaltene Endprodukt in ein physiologisch unbedenkliches Säureadditionssalz überführt.

3. Verfahren zur Herstellung von substituierten 2-Phenylamino-imidazolinen-(2) der in Anspruch 1 aufgeführten Formel (I), worin $R_1$ und $R_2$ Brom und R $—CH_2—CH{=}CH_2$, $—CH_2—CH{=}CH—CH_3$, oder

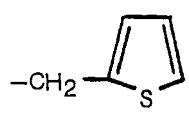

bedeutet und von deren physiologisch verträglichen Säureadditionssalzen, dadurch gekennzeichnet, daß man ein 2-(N-Phenyl-N-hydroxy-amino)-imidazolin-(2) der in Anspruch 1 aufgeführten Formel (II), worin $R_1$ und $R_2$ die oben genannten Bedeutungen haben, oder dessen Natriumsalz mit einem Halogenid der allgemeinen Formel (III)

Hal—R III

worin Hal ein Chlor-, Brom- oder Jodatom bedeutet und R die oben angeführten Bedeutungen hat, umsetzt und daß man gegebenenfalls das erhaltene Endprodukt in ein physiologisch unbedenkliches Säureadditionssalz überführt.

4. Verfahren zur Herstellung des substituierten 2-Phenylamino-imidazolins-(2) der in Anspruch 1 aufgeführten Formel I, worin $R_1$ und $R_2$ Chlor und R

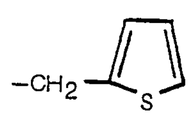

bedeutet und von dessen physiologisch verträglichen Säureadditionssalzen, dadurch gekennzeichnet, daß man das 2-(N-Phenyl-N-hydroxy-amino)-imidazolin-(2) der in Anspruch 1 aufgeführten Formel (II), worin $R_1$ und $R_2$ die oben genannten Bedeutungen haben, oder dessen Natriumsalz mit einem Halogenid der allgemeinen Formel (III)

Hal—R III

worin Hal ein Chlor-, Brom- oder Jodatom bedeutet und R die oben angeführte Bedeutung hat, umsetzt und daß man gegebenenfalls das erhaltene Endprodukt in ein physiologisch unbedenkliches Säureadditionssalz überführt.

5. Verfahren zur Herstellung des substituierten 2-Phenylamino-imidazolins-(2) der in Anspruch 1

aufgeführten Formel (I), worin $R_1$ und $R_2$ Fluor und R $-CH_2-CH=CH_2$ bedeutet und von dessen physiologisch verträglichen Säureadditionssalzen, dadurch gekennzeichnet, daß man das 2-(N-Phenyl-N-hydroxy-amino)-imidazolin-(2) der in Anspruch 1 aufgeführten Formel (II), worin $R_1$ und $R_2$ die oben genannten Bedeutungen haben, oder dessen Natriumsalz mit einem Halogenid der allgemeinen Formel (III)

$$Hal-CH_2-CH=CH_2 \qquad III$$

worin Hal ein Chlor-, Brom- oder Jodatom bedeutet, umsetzt und daß man gegebenenfalls das erhaltene Endprodukt in ein physiologisch unbedenkliches Säureadditionssalz überführt.

6. Verfahren zur Herstellung von Arzneimitteln, dadurch gekennzeichnet, daß man eine oder mehrere Verbindungen der allgemeinen Formel I oder deren physiologisch verträglichen Säureadditionssalze nach Anspruch 1 bis 5 mit überlichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

7. Verwendung von Verbindungen der allgemeinen Formel I oder deren physiologisch verträglichen Säureadditionssalzen nach Anspruch 1 bis 5 zur Herstellung von Arzneimitteln.

**Revendications pour les Etats contractants: BE CH DE FR GB IT LI LU NL SE**

1. 2-phénylamino-imidazolines-(2) substituées de formule générale (I)

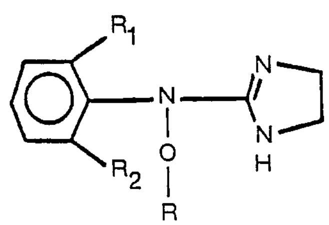

I

dans laquelle $R_1$ représente le brome, $R_2$ le fluor et R

$-CH_2-CH=CH_2$, $-CH_2-CH=CH-CH_3$, $-CH_2-CH_2-CH=CH_2$ ou

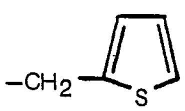

ainsi que leurs sels d'addition d'acides physiologiquement supportables.

2. Composés de formule (I) indiquée dans la revendication 1, dans laquelle $R_1$ représente le fluor, $R_2$ trifluorométhyle et

R $-CH_2-CH=CH_2$, $-CH_2-CH=CH-CH_3$, $-CH_2-C_6-H_5$, $-CH_3$, $-C_2H_5$, $-nC_3H_7$, $-nC_4H_9$,

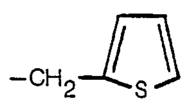 ou 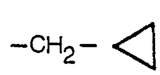

ainsi que leurs sels d'addition d'acides physiologiquement supportables.

3. Composés de formule (I) indiquée dans la revendication 1, dans laquelle $R_1$ et $R_2$ représentent le brome et R représente $CH_2-CH=CH_2$, $-CH_2-CH=CH-CH_3$ ou

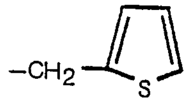

ainsi que leurs sels d'addition d'acides physiologiquement supportables.

4. Composé de formule (I) indiquée dans la revendication 1, dans laquelle $R_1$ et $R_2$ représentent le chlore et R représente

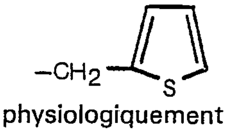

ainsi que ses sels d'addition d'acides physiologiquement supportables.

5. Composé de formule (I) indiquée dans la revendication 1, dans laquelle $R_1$ et $R_2$ représentent le fluor et R représente $—CH_2—CH{=}CH_2$ ainsi que ses sels d'addition d'acides physiologiquement supportables.

6. Procédé pour la préparation de 2-phénylamino-imidazolines selon la revendication 1 à 5, caractérisé en ce que

on fait réagir une 2-(N-phényl-N-hydroxy-amino)-imidazoline-(2) de formule générale (II)

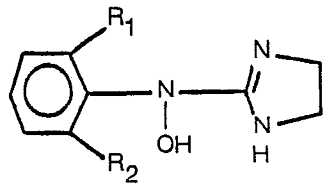

II

dans laquelle $R_1$ et $R_2$ ont les significations mentionnées dans les revendications 1 à 5, ou son sel de sodium avec un halogénure de formule générale (III)

$$Hal—R \qquad III$$

dans laquelle Hal représente un atome de chlore, de brome ou d'iode et R a les significations indiquées dans les revendications 1 à 5 et en ce qu'éventuellement on transforme le produit final obtenu en un sel d'addition d'acide physiologiquement supportable.

7. Préparations pharmaceutiques, caractérisées en ce qu'elles contiennent, en tant que substance active, un ou plusieurs composés selon la revendication 1 à 5.

8. Procédé pour la préparation de médicaments selon la revendication 7, caractérisé en ce qu'on formule un ou plusieurs composés selon la revendication 1 à 5 avec des adjuvants, excipients, désagrégeants ou lubrifiants ou respectivement substances pour obtenir un effet retard galéniques habituels en comprimés, capsules, suppositoires, solutions ou poudres.

9. Composés selon la revendication 1 à 5 pour l'utilisation dans la lutte contre les maladies du coeur et des coronaires.

**Revendications pour l'Etat contractant: AT**

1. Procédé pour la préparation de 2-phénylamino-imidazolines-(2) substituées de formule générale (I)

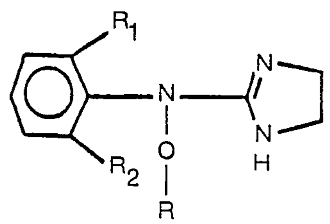

I

dans laquelle $R_1$ représente la brome, $R_2$ le fluor et

R $—CH_2—CH{=}CH_2$, $—CH_2—CH{=}CH—CH_3$, $—CH_2—CH_2—CH{=}CH_2$

ou

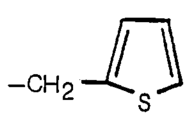

ainsi que de leurs sels d'addition d'acides physiologiquement supportables, caractérisé en ce qu'on fait réagir une 2-(N-phényl-N-hydroxyamino)-imidazoline-(2) de formule générale (II)

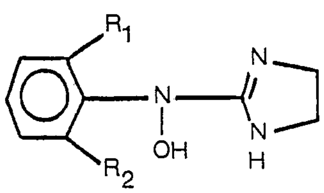

II

dans laquelle $R_1$ et $R_2$ ont les significations mentionnées plus haut, ou son sel de sodium avec un halogénure de formule générale (III)

Hal—R III

dans laquelle Hal représente un atome de chlore, de brome ou d'iode et R a les significations indiquées plus haut et en ce qu'éventuellement on transforme le produit final obtenu en un sel d'addition d'acide physiologiquement supportable.

2. Procédé pour la préparation de 2-phénylamino-imidazolines-(2) substituées de formule (I) indiquée dans la revendication 1, dans laquelle $R_1$ représente le fluor, $R_2$ trifluorométhyle et R représente:

$—CH_2—CH=CH_2$, $CH_2—CH=CH—CH_3$, $—CH_2—C_6H_5$, $—CH_3$, $—C_2H_5$, $-nC_3H_7$, $-nC_4H_9$,

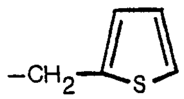 ou 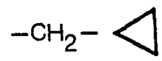

et de leurs sels d'addition d'acides physiologiquement supportables, caractérisé en ce qu'on fait réagir une 2-(N-phényl-N-hydroxy-amino)-imidazoline-(2) de formule (II) indiquée dans la revendication 1 dans laquelle $R_1$ et $R_2$ ont les significations mentionnées plus haut, ou son sel de sodium avec un halogénure de formule générale (III)

Hal—R III

dans laquelle Hal représente un atome de chlore, de brome ou d'iode et R a les significations indiquées plus haut et en ce qu'éventuellement on transforme le produit final obtenu en un sel d'addition d'acide physiologiquement inoffensif.

3. Procédé pour la préparation de 2-phénylamino-imidazolines-(2) substituées de formule (I) indiquée dans la revendication 1, dans laquelle $R_1$ et $R_2$ représentent le brome et R représente $—CH_2—CH=CH_2$, $—CH_2—CH=CH—CH_3$, ou

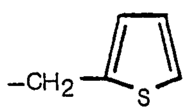

et de leurs sels d'addition d'acides physiologiquement supportables, caractérisé en ce qu'on fait réagir une 2-(N-phényl-N-hydroxy-amino)-imidazoline-(2) de formule (II) indiquée dans la revendication 1, dans laquelle $R_1$ et $R_2$ ont les significations indiquées plus haut, ou son sel de sodium avec un halogénure de formule générale (III)

Hal—R III

dans laquelle Hal représente un atome de chlore, de brome ou d'iode et R a les significations indiquées plus haut et en ce qu'éventuellement on transforme le produit final obtenu en un sel d'addition d'acide physiologiquement inoffensif.

4. Procédé pour la préparation de la 2-phénylamino-imidazoline-(2) substituée de formule (I) indiquée dans la revendication 1, dans laquelle $R_1$ et $R_2$ représentent le chlore et R représente

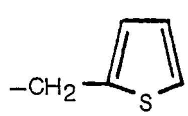

et de ses sels d'addition d'acides physiologiquement supportables, caractérisé en ce qu'on fait réagir la 2-(N-phényl-N-hydroxy-amino)-imidazoline-(2) de formule (II) indiquée dans la revendication 1, dans laquelle $R_1$ et $R_2$ ont les significations mentionnées plus haut, ou son sel de sodium avec un halogénure de formule générale (III)

Hal—R III

dans laquelle Hal représente un atome de chlore, de brome ou d'iode et R a la signification indiquée plus haut et en ce qu'éventuellement on transforme le produit final obtenu en un sel d'addition d'acide physiologiquement inoffensif.

5. Procédé pour la préparation de la 2-phénylamino-imidazoline-(2) substituée de formule (I) indiquée dans la revendication 1, dans laquelle $R_1$ et $R_2$ représentent le fluor et R représente $—CH_2—CH=CH_2$ et de ses sels d'addition d'acides physiologiquement supportables, caractérisé en ce qu'on fait réagir la 2-(N-phényl-N-hydroxy-amino)-imidazoline-(2) de formule (II) indiquée dans la revendication 1, dans laquelle $R_1$ et $R_2$ ont les significations mentionnées plus haut, ou son sel de sodium avec un halogénure de formule générale (III)

$$Hal—CH_2—CH=CH_2 \qquad III$$

dans laquelle Hal représente un atome de chlore, de brome ou d'iode et en ce qu'éventuellement on transforme le produit final obtenu en un sel d'addition d'acide physiologiquement inoffensif.

6. Procédé pour la préparation de médicaments, caractérisé en ce qu'on formule un ou plusieurs composés de formule générale (I) ou leurs sels d'addition d'acides physiologiquement supportables selon la revendication 1 à 5 avec des adjuvants, excipients, désagrégeants ou diluants ou respectivement substances pour obtenir un effect retard galéniques, habituels en comprimés, capsules, suppositoires, solutions ou poudres.

7. Utilisation des composés de formule générale (I) ou de leurs sels d'addition d'acides physiologiquement supportables selon la revendication 1 à 5 pour la préparation de médicaments.

**Claims for the Contracting States: BE CH DE FR GB IT LI LU NL SE**

1. Substituted 2-Phenylamino-imidazolines-(2) of general formula (I)

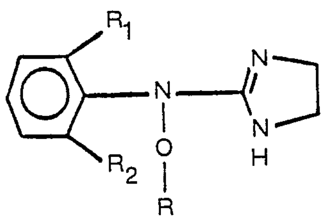

I

in which $R_1$ represents bromine, $R_2$ represents fluorine and R represents

$$—CH_2—CH=CH_2,\ —CH_2—CH=CH—CH_3,\ —CH_2—CH_2—CH=CH_2$$

or

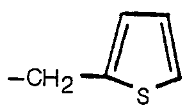

and their physiologically compatible acid addition salts.

2. Compounds of formula (I) mentioned in claim 1, in which $R_1$ represents fluorine, $R_2$ represents trifluormethyl and R represents

$$—CH_2—CH=CH_2,\ —CH_2—CH=CH—CH_3,\ CH_2—C_6H_5,\ —CH_3,\ —C_2H_5,\ -nC_3H_7,\ -nC_4H_9,$$

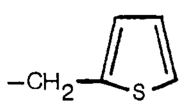 or 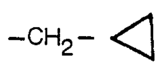

and their physiologically compatible acid addition salts.

3. Compounds of formula (I) mentioned in claim 1, in which $R_1$ and $R_2$ represent bromine and R represents $—CH_2—CH=CH_2$, $—CH_2—CH=CH—CH_3$ or

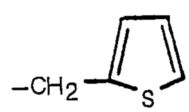

and their physiologically compatible acid addition salts.

4. Compound of formula (I) mentioned in claim 1, in which $R_1$ and $R_2$ represent chlorine and R represents

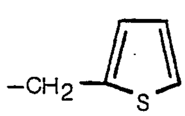

and its physiologically compatible acid addition salts.

5. Compound of formula (I) mentioned in claim 1, in which $R_1$ and $R_2$ represent fluorine and R represents $—CH_2—CH=CH_2$, and its physiologically compatible acid addition salts.

6. Process for preparing substituted 2-Phenylamino-imidazolines according to claims 1—5, characterised in that a 2-(N.Phenyl-N-hydroxy-amino)-imidazoline-(2) of general formula (II)

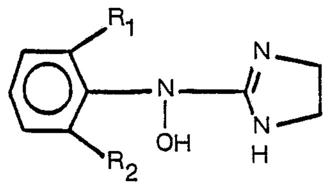

II

in which $R_1$ and $R_2$ have the meanings mentioned in claims 1—5, or its sodium salt is reacted with a halide of general formula (III)

HAL—R III

in which HAL represents a chlorine, bromine or iodine atom and R has the meanings mentioned in claims 1—5, and in that, if appropriate, the final product obtained is converted into a physiologically compatible acid addition salt.

7. Pharmaceutical preparations, characterised in that they contain as an active substance one or more compounds according to claims 1—5.

8. Process for preparing pharmaceuticals according to claim 7, characterised in that one or more compounds according to claims 1—5 are formulated with conventional galenic excipients, carriers, boosting agents, lubricants or substances for achieving sustained release into tablets, capsules, suppositories, solutions or powders.

9. Compounds according to claims 1—5 for use in combating heart and coronary diseases.

**Claims for the Contracting State: AT**

1. Process for preparing substituted 2-Phenylamino-imidazolines-(2) of general formula (I)

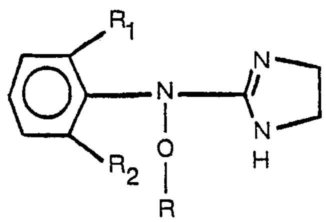

I

in which $R_1$ represents bromine, $R_2$ represents fluorine and R represents

$-CH_2-CH=CH_2$, $-CH_2-CH=CH-CH_3$, $-CH_2-CH_2-CH=CH_2$ or

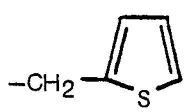

and their physiologically compatible acid addition salts, characterised in that a 2-(N-Phenyl-N-hydroxyamino)-imidazoline-(2) of general formula (II)

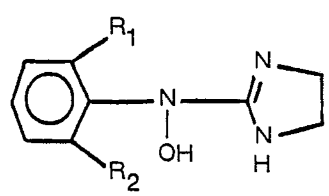

II

in which $R_1$ and $R_2$ have the meanings mentioned above, or its sodium salt is reacted with a halide of general formula (III)

HAL—R III

in which HAL represents a chlorine, bromine or iodine atom and R has the meanings mentioned above, and in that, if appropriate, the final product obtained is converted into a physiologically compatible acid addition salt.

2. Process for preparing substituted 2-Phenylamino-imidazolines-(2) of formula (I) mentioned in claim 1, in which $R_1$ represents fluorine, $R_2$ represents trifluormethyl and R represents

$-CH_2-CH=CH_2$, $-CH_2-CH=CH-CH_3$, $-CH_2-C_6H_5$, $-CH_3$, $-C_2H_5$, $-nC_3H_7$, $-nC_4H_9$,

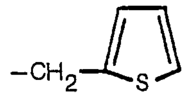 or 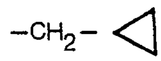

and their physiologically compatible acid addition salts, characterised in that a 2-(N-Phenyl-hydroxyamino)-imidazoline-(2) of formula (III) mentioned in claim 1, in which $R_1$ and $R_2$ have the meanings mentioned above, or its sodium salt is reacted with a halide of general formula (III)

$$HAL-R \qquad III$$

in which HAL represents a chlorine, bromine or iodine atom and R has the meanings mentioned above, and in that, if appropriate, the final product obtained is converted into a physiologically harmless acid addition salt.

3. Process for preparing substituted 2-Phenylamino-imidazolines-(2) of formula (I) mentioned in claim 1, in which $R_1$ and $R_2$ represent bromine and R represents

$-CH_2-CH=CH_2$, $-CH_2-CH=CH-CH_3$ or 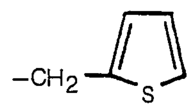

and their physiologically compatible acid addition salts, characterised in that a 2-(N-Phenyl-N-hydroxyamino)-imidazoline-(2) of formula (II) mentioned in claim 1, in which $R_1$ and $R_2$ have the meanings mentioned above, or its sodium salt is reacted with a halide of general formula (III)

$$HAL-R \qquad III$$

in which HAL represents a chlorine, bromine or iodine atom and R has the meanings mentioned above, and in that, if appropriate, the final product obtained is converted into a physiologically harmless acid addition salt.

4. Process for preparing the substituted 2-Phenylamino-imidazoline-(2) of formula (I) mentioned in claim 1, in which $R_1$ and $R_2$ represent chlorine and R represents

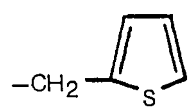

and its physiologically compatible acid addition salts, characterised in that the 2-(N-Phenyl-hydroxyamino)-imidazoline-(2) of formula (II) mentioned in claim 1, in which $R_1$ and $R_2$ have the meanings mentioned above, or its sodium salt is reacted with a halide of general formula (III)

$$HAL-R \qquad III$$

in which HAL represents chlorine, bromine or iodine atom and R has the meaning mentioned above, and in that, if appropriate, the final product obtained is converted into a physiologically harmless acid addition salt.

5. Process for preparing the substituted 2-Phenylamino-imidazoline-(2) of formula (I) mentioned in claim 1, in which $R_1$ and $R_2$ represent fluorine and R represents $-CH_2-CH=CH_2$, and its physiologically compatible acid addition salts, characterised in that the 2-(N-Phenyl-N-hydroxyamino)-imidazoline-(2) of formula (II) mentioned in claim 1, in which $R_1$ and $R_2$ have the meanings mentioned above, or its sodium salt is reacted with a halide of general formula (III)

$$HAL-CH_2-CH=CH_2 \qquad III$$

in which HAL represents a chlorine, bromine or iodine atom, and in that, if appropriate, the final product obtained is converted into a physiologically harmless acid addition salt.

6. Process for preparing pharmaceuticals, characterised in that one or more compounds of general formula (I) or their physiologically compatible acid addition salts according to claims 1—5 are formulated with conventional galenic excipients, carriers, boosting agents, lubricants or substances for achieving sustained release into tablets, capsules, suppositories, solutions or powders.

7. Use of compounds of general formula (I) or their physiologically compatible acid addition salts according to claims 1—5 for preparing pharmaceuticals.